# EUROPÄISCHE PATENTSCHRIFT

(11) **EP 0 668 329 B1**
(45) Veröffentlichungstag und Bekanntmachung des Hinweises auf die Patenterteilung: **22.07.1998**
(21) Anmeldenummer: 95101864.7
(22) Anmeldetag: 11.02.1995
(51) Int. Cl.: C09C 1/00, C09D 7/12, C09D 11/00, C08K 9/02, C03C 4/02, C04B 33/14, A61K 7/00

(54) **Mehrfach beschichtete metallische Glanzpigmente**
Brilliant pigments with multiple coatings
Pigments brillants à revêtements multiples

(30) Priorität: 21.02.1994 DE 4405492; 30.08.1994 US 297857
(43) Veröffentlichungstag der Anmeldung: 23.08.1995
(73) Patentinhaber: BASF Aktiengesellschaft, 67063 Ludwigshafen (DE)
(72) Erfinder: Schmid, Raimund, Dr., D-67435 Neustadt (DE); Mronga, Norbert, Dr., D-69221 Dossenheim (DE)

(56) Entgegenhaltungen:
- EP-A- 0 571 836
- DE-A- 4 241 753
- DE-A- 4 319 669

## Beschreibung

Die vorliegende Erfindung betrifft neue Glanzpigmente auf der Basis von mehrfach beschichteten plättchenförmigen metallischen Substraten mit
A) einer ersten, im wesentlichen aus Siliciumoxid, Siliciumoxidhydrat, Aluminiumoxid und/oder Aluminiumoxidhydrat bestehenden Schicht,
B) einer zweiten, im wesentlichen aus Metall und/oder nichtselektiv absorbierendem Metalloxid bestehenden Schicht und
C) gewünschtenfalls einer dritten, im wesentlichen aus farblosem oder selektiv absorbierendem Metalloxid bestehenden Schicht,
erhältlich durch
a) naßchemische Belegung der Substratteilchen mit Siliciumoxid, Siliciumoxidhydrat, Aluminiumoxid und/oder Aluminiumoxidhydrat durch hydrolytische Zersetzung von organischen Silicium- und/oder Aluminiumverbindungen, bei denen die organischen Reste über Sauerstoffatome an die Metalle gebunden sind, in Gegenwart eines organischen Lösungsmittels, in welchem die Metallverbindungen löslich sind, und gegebenenfalls anschließende Trocknung,
b) weitere Belegung der in Schritt a) erhaltenen Teilchen mit Metall durch
   b1) Gasphasenzersetzung flüchtiger Metallverbindungen in einer inerten Atmosphäre oder
   b2) stromlose, naßchemische Metallabscheidung und gegebenenfalls anschließende Trocknung
   oder mit nichtselektiv absorbierendem Metalloxid durch
   b3) Gasphasenzersetzung flüchtiger Metallverbindungen in Gegenwart von Sauerstoff und/oder Wasserdampf und
c) gewünschtenfalls zusätzliche Belegung der in Schritt b) erhaltenen Teilchen mit farblosem oder selektiv absorbierendem Metalloxid durch
   c1) Gasphasenzersetzung flüchtiger Metallverbindungen in Gegenwart von Sauerstoff und/oder Wasserdampf oder
   c2) naßchemische Belegung durch hydrolytische Zersetzung organischer Metallverbindungen, bei denen die organischen Reste über Sauerstoffatome an die Metalle gebunden sind, in Gegenwart eines organischen Lösungsmittels, in welchem die Metallverbindungen löslich sind, und anschließende Trocknung.

Außerdem betrifft die Erfindung Mischungen dieser Pigmente (I) mit mehrfach beschichteten silikatischen Plättchen (II), die
A') eine erste, im wesentlichen aus farblosem oder selektiv absorbierendem Metalloxid bestehende Schicht,
B') eine zweite, im wesentlichen aus Metall und/oder nichtselektiv absorbierendem Metalloxid bestehende Schicht und
C') gewünschtenfalls eine dritte, im wesentlichen aus farblosem oder selektiv absorbierendem Metalloxid bestehende Schicht
aufweisen, als wesentlichen Komponenten.

Weiterhin betrifft die Erfindung die Herstellung dieser Pigmente sowie ihre Verwendung zum Einfärben von Lacken, Druckfarben, Tinten, Kunststoffen, Gläsern und keramischen Produkten.

Glanz- oder Effektpigmente werden in vielen Bereichen der Technik eingesetzt, beispielsweise in Automobillacken, in der dekorativen Beschichtung, der Kunststoffeinfärbung, in Anstrich-, Druck-, insbesondere Sicherheitsdruckfarben sowie in der Kosmetik.

Ihre optische Wirkung beruht auf der gerichteten Reflexion von Licht an überwiegend flächig ausgebildeten, zueinander parallel ausgerichteten, metallischen oder stark lichtbrechenden Pigmentteilchen. Je nach Zusammensetzung der Pigmentplättchen erzeugen Interferenz-, Reflexions- und Absorptionsphänomene winkelabhängige Farb- und Helligkeitseindrücke.

Aufgrund ihrer nicht kopierbaren optischen Effekte gewinnen diese Pigmente zunehmende Bedeutung für die Herstellung von fälschungssicheren Wertschriften, wie Geldscheinen, Schecks, Scheckkarten, Kreditkarten, Steuermarken, Briefmarken, Bahn- und Flugtickets, Telefonkarten, Lotterielosen, Geschenkzertifikaten, Ausweisen und Identifikationskarten.

Kennzeichnungen, die mit den Effektpigmenten angefertigt wurden, und das Fehlen dieser Kennzeichnungen oder ihre Veränderung, beispielsweise in einer Farbkopie (Verschwinden von Farbflops und Glanzeffekten), sind ohne Hilfsmittel mit bloßem Auge sicher erkennbar und ermöglichen so eine leichte Unterscheidung des Originals von der Kopie.

Aus den US-A-3 438 796, 4 879 140 und 5 059 245 sind alternierende Metall- und Metalloxidschichten aufweisende Effektpigmente bekannt, die sich jedoch aufgrund ihrer Herstellung von den erfindungsgemäßen Pigmenten unterscheiden.

Die dort beschriebenen Pigmente werden durch physikalische Techniken wie Aufdampfen im Hochvakuum auf ein Substrat, das mit einer auflösbaren Beschichtung versehen sein kann oder selbst auflösbar ist, Abtrennung vom Substrat und anschließende Zerkleinerung oder durch Plasmazersetzung, nachfolgendes Abblättern des Zersetzungsprodukts von den Reaktorwänden und Ausblasen hergestellt.

Bei den auf diese Weise erhaltenen Pigmenten ist eine gewissermaßen als Substrat wirkende mittlere Schicht im Gegensatz zu den Substratteilchen im erfindungsgemäßen Fall nicht vollständig durch die weiteren Schichten umhüllt. An den bei der Zerkleinerung entstehenden Grenzflächen ist der Schichtaufbau zu erkennen, und es liegt dort keine äußere Begrenzungsschicht vor, was auch zu Instabilitäten, z.B. gegenüber wäßrigen Systemen führen kann.

Zudem sind diese Herstellungsverfahren sehr kostspielig und zeitaufwendig, weshalb die so hergestellten Pigmente nur in kleinen Mengen zur Verfügung stehen.

In der EP-A-571 836 werden metalloxid- und metallbeschichtete metallische Interferenzpigmente beschrieben, die durch Gasphasenbeschichtung hergestellt werden.

Schließlich ist aus der US-A-2 885 366 die Beschichtung vom Aluminiumplättchen mit Siliciumoxid aus Wasserglaslösungen bekannt. Die Oxidschicht wird hier jedoch nur zur Passivierung der Aluminiumoberfläche aufgebracht.

Der Erfindung lag die Aufgabe zugrunde, besonders farbstarke Metalleffektpigmente bereitzustellen, die auf wirtschaftliche Weise hergestellt werden können.

Demgemäß wurden die eingangs definierten Glanzpigmente und ihre Mischungen mit mehrfach beschichteten silikatischen Plättchen gefunden.

Als besonders bevorzugte Variante wurden Glanzpigmente auf der Basis von mehrfach beschichteten, im wesentlichen aus Aluminium bestehenden, plättchenförmigen Substraten mit
A) einer ersten, 50 bis 600 nm dicken, im wesentlichen aus Siliciumoxid und/oder Siliciumoxidhydrat bestehenden Schicht,
B) einer zweiten, 1 bis 25 nm dicken, im wesentlichen aus Molybdän, Chrom, Wolfram und/oder Eisen bestehenden Schicht und
C) gewünschtenfalls einer dritten, 5 bis 50 nm dicken, im wesentlichen aus Siliciumoxid, Siliciumoxidhydrat, Aluminiumoxid und/oder Aluminiumoxidhydrat bestehenden Schicht
gefunden.

Weiterhin wurde ein Verfahren zur Herstellung der Glanzpigmente gefunden, welches dadurch gekennzeichnet ist, daß man die metallischen Substratteilchen zunächst durch
a) hydrolytische Zersetzung von organischen Silicium- und/oder Aluminiumverbindungen, bei denen die organischen Reste über Sauerstoffatome an die Metalle gebunden sind, in Gegenwart eines organischen Lösungsmittels, in welchem die Metallverbindungen löslich sind, naßchemisch mit Siliciumoxid, Aluminiumoxid und/oder Aluminiumoxidhydrat belegt und gegebenenfalls anschließend trocknet, dann durch
b1) Gasphasenzersetzung flüchtiger Metallverbindungen in einer inerten Atmosphäre oder
b2) stromlose, naßchemische Metallabscheidung und gegebenenfalls anschließende Trocknung mit Metall oder durch
b3) Gasphasenzersetzung flüchtiger Metallverbindungen in Gegenwart von Sauerstoff und/oder Wasserdampf mit nichtselektiv absorbierendem Metalloxid belegt und gewünschtenfalls zusätzlich durch
c1) Gasphasenzersetzung flüchtiger Metallverbindungen in Gegenwart von Sauerstoff und/oder Wasserdampf oder durch
c2) hydrolytische Zersetzung organischer Metallverbindungen, bei denen die organischen Reste über Sauerstoffatome an die Metalle gebunden sind, in Gegenwart eines organischen Lösungsmittels, in welchem die Metallverbindungen löslich sind, und anschließende Trocknung mit farblosem oder selektiv absorbierendem Metalloxid belegt.

Nicht zuletzt wurde die Verwendung dieser Glanzpigmente und Glanzpigmentmischungen zur Einfärbung von Lacken, Druckfarben, Tinten, Kunststoffen, Gläsern, keramischen Produkten und Zubereitungen der dekorativen Kosmetik gefunden.

Für die erfindungsgemäßen Pigmente sind als Substrat alle für Metalleffektpigmente bekannten Metalle und Legierungen in Plättchenform geeignet. Z.B. kommen neben Stahl, Kupfer und seinen Legierungen wie Messing und Bronzen vor allem Aluminium und seine Legierungen wie Aluminiumbronze in Betracht.

Bevorzugt sind Aluminiumflakes, die in einfacher Weise durch Herausstanzen aus Aluminiumfolie oder nach gängigen Verdüsungs- und Mahltechniken herzustellen sind.

So sind beispielsweise Aluminiumpigmente geeignet, die nach dem sogenannten Hall-Verfahren in Testbenzin durch Naßmahlung hergestellt werden. Ausgangsmaterial ist ein atomisierter, spratziger Aluminiumgrieß, welcher in Kugelmühlen in Testbenzin und in Gegenwart eines Schmiermittels zu plättchenförmigen Teilchen verformt bzw. zerkleinert und anschließend klassiert wird.

Es können handelsübliche Produkte eingesetzt werden. Jedoch sollte die Oberfläche der Aluminiumteilchen weitgehend frei von Fetten oder anderen Belegmitteln sein. Diese Substanzen können zum Teil durch Lösungsmittelbehandlung oder besser, wie in der DE-A-42 23 384 beschrieben, durch oxidative Behandlung entfernt werden.

Weiterhin können die metallischen Substratteilchen passiviert sein, d.h. insbesondere gegenüber Wasser stabilisierende Beschichtungen aufweisen. Beispielhaft sei hier auf Farbe + Lack 97, 4/1991, S. 311-314 und die dort zitierte Literatur sowie auf die DE-A- 42 36 332 hingewiesen.

Als passivierende Beschichtungen sollen dabei auch Metalloxidschichten verstanden werden. Beispiele für weitere geeignete Substrate sind daher eisenoxidbeschichtete Metallpigmente (z.B. EP-A-33 457) mit goldener bis roter Eigenfarbe und zart pastellfarben titandioxidbeschichtete Metallpigmente (z.B. EP-A-338 428).

Die Größe der Substratteilchen ist an sich nicht kritisch und kann auf den jeweiligen Anwendungszweck abgestimmt werden. In der Regel haben die Teilchen mittlere größte Durchmesser von etwa 1 bis 200 µm, insbesondere etwa 5 bis 100 µm, und Dicken von etwa 0,1 bis 5 µm, insbesondere um etwa 0,5 µm. Ihre spezifische freie Oberfläche (BET) beträgt im allgemeinen 0,1 bis 5 m²/g.

Die erfindungsgemäßen Glanzpigmente zeichnen sich durch eine Mehrfachbeschichtung des metallischen Substrats aus.

Die erste Schicht (A) ist aus Aluminiumoxid, Aluminiumoxidhydrat und bevorzugt Siliciumoxid, Siliciumoxidhydrat sowie auch aus deren Mischungen aufgebaut.

Die Dicke der Schicht (A) beträgt im allgemeinen 1 bis 800 nm, bevorzugt 50 bis 600 nm. Da die Schicht (A) im wesentlichen den Farbton der erfindungsgemäßen Pigmente bestimmt, hat die Schicht (A) für die ein besonders ausgeprägtes Farbenspiel zeigenden und daher auch bevorzugten erfindungsgemäßen Glanzpigmente eine Mindestschichtdicke von etwa 70 nm.

Mit wachsender Schichtdicke von (A) durchläuft man bei den mit der Schicht (A) und der schwarzen Schicht (B) belegten Pigmenten bei einem Betrachtungswinkel von 25° mehrmals nacheinander die Interferenzfarben blau, grün, gold, rot. Die Winkelabhängigkeit des Farbtons nimmt von der ersten Interferenzfarbenserie nach höheren Serien (also dicker werdenden Schichten (A)) zu. So kippt beispielsweise ein rötlicher Goldton der ersten Serie winkelabhängig ab in ein grünliches Gold, während ein solcher Farbton aus der zweiten oder dritten Interferenzserie in die Komplementärfarbe, ein grünstichiges Blau, umschlägt.

Die zweite, nichtseleketiv absorbierende Schicht (B) besteht im wesentlichen aus Metallen, bevorzugt solchen, die durch Gasphasenzersetzung flüchtiger Verbindungen aufgebracht werden können, wie vor allem Molybdän, Wolfram, Chrom, auch Cobalt und Nickel oder Gemische dieser Metalle, oder schwarzen Metalloxiden wie vor allem Magnetit, auch Nickeloxid, Cobaltoxid (CoO, Co₃O₄) und Vanadiumoxid (VO₂, V₂O₃) sowie deren Mischungen, beispielsweise Eisen und Magnetit.

Weiterhin sind auch solche Metalle, die sich naßchemisch durch Reduktion aus Metallsalzlösungen abscheiden lassen, für die Schicht (B) geeignet. Als Beispiele seien Silber, Kupfer, Gold, Palladium und Platin sowie auch Cobalt und Nickel und Legierungen wie NiP, NiB, NiCo, NiWP, CoP und AgAu genannt.

Die schwarze Schicht (B) darf selbstverständlich nicht deckend sein, sondern muß für Licht teilweise durchlässig sein. Auf diese Weise senkt sie den Weißsockel des auftreffenden und reflektierenden Lichtes ab und bewirkt so eine Verstärkung der kaum sichtbaren Interferenzfarbe des mit Metalloxid beschichteten Substrates.

Je nach den optischen Eigenschaften des Schichtmaterials (B) betragen die Schichtdicken im allgemeinen 1 bis 100 nm. Bei stark absorbierenden, hochbrechenden Materialien wie Molybdän oder Chrom ist in der Regel eine Schichtdicke von 1 bis 25 nm ausreichend, um den gewünschten Effekt einzustellen. Schwächer absorbierende oder niedriger brechende Materialien wie Magnetit erfordern dickere Schichten von etwa 10 bis 50 nm.

Weiterhin können die erfindungsgemäßen Glanzpigmente noch eine dritte Schicht (C) aufweisen, die aus farblosen oder selektiv absorbierenden Metalloxiden aufgebaut ist. Bevorzugt sind z.B. Aluminiumoxid, -oxidhydrat, Zirkon-, Titan-, Zinn-, Eisen- und Chromoxid und besonders bevorzugt Siliciumoxid und -oxidhydrat. Diese Deckschicht bewirkt insbesondere bei metallischen Schichten (B) eine deutliche Verbesserung der Beständigkeit gegen Umwelteinflüsse.

Die Dicke der Schicht (C) ist an sich nicht kritisch, im allgemeinen beträgt sie etwa 1 bis 400 nm, insbesondere 5 bis 250 nm.

Selbstverständlich kann auch die Schicht (C) zur Interferenz des Pigmentes beitragen und dabei die Interferenzreihe an der durch das mit (A) und (B) beschichtete Substrat bestimmten Stelle fortsetzen. Dies ist beispielsweise der Fall, wenn Zirkon- oder Titanoxid als Schicht (C) aufgebracht werden. Besteht dagegen die Schicht (C) im wesentlichen aus Siliciumoxid, so wird sich diese Schicht im Anwendungsmedium (z.B. Lacken, Druckfarben oder Tinten), das einen ähnlichen Brechungsindex aufweist, koloristisch kaum bemerkbar machen.

Farbige Metalloxide wie Eisen- und Chromoxid werden schließlich die Interferenzfarbe des Mehrschichtsystems durch Beimischen ihrer Absorptionsfarbe modifizieren und mit zunehmender Schichtdicke schließlich überdecken.

Eine aufgrund ihrer hohen Farbstärke besonders bevorzugte Ausführungsform der erfindungsgemäßen Glanzpigmente stellen mit (A) Siliciumdioxid und/oder Silicumoxidhydrat, (B) Molybdän, Chrom, Wolfram und/oder Eisen und gewünschtenfalls wiederum Siliciumdioxid, Siliciumoxidhydrat, Aluminiumoxid und/oder Aluminiumoxidhydrat (C) beschichtete Aluminiumplättchen dar, wobei die Dicken der einzelnen Schichten vorzugsweise 50 bis 600 nm (A), 1 bis 25 nm, insbesondere 1 bis 20 nm (B) und 5 bis 250 nm (C) betragen. Ganz besonders bevorzugt sind die Glanzpigmente mit einer im wesentlichen aus Molybdän bestehenden Schicht (B).

Insgesamt zeichnen sich bei den erfindungsgemäßen Glanzpigmenten alle Schichten durch ihren gleichmäßigen, homogenen und filmartigen Aufbau und ihre Fähigkeit zur Interferenz auch der dickeren Schichten aus, so daß kräftige Interferenzfarben zeigende Mehrschichtsysteme entstehen.

Aus koloristischen Gründen sind Mischungen der erfindungsgemäßen metallischen Pigmente (I) mit ebenfalls mehrfach beschichteten silikatischen Plättchen (II) von besonderem Interesse.

Als silikatische Substrate kommen dabei insbesondere helle bzw. weiße Glimmer in Betracht, wobei Schuppen von vorzugsweise naß vermahlenem Muskovit besonders bevorzugt sind. Selbstverständlich sind auch andere natürliche Glimmer wie Phlogopit und Biotit, künstliche Glimmer, Talk- und Glasschuppen geeignet.

Die zum Einsatz kommenden silikatischen Substratteilchen weisen eine Metalloxidschicht (A') auf, die vorzugsweise aus hochbrechenden Metalloxiden wie Titan-, Zirkon-, Zink-, Zinn-, Chrom-, Eisenoxid und/oder Bismutoxychlorid aufgebaut ist. Aluminium- und Siliciumoxid können ebenfalls enthalten sein.

Besonders bevorzugt sind Glimmerpigmente, die eine im wesentlichen aus Titandioxid bestehende und die weiteren genannten Oxide höchstens in untergeordneter Menge enthaltende Schicht (A') aufweisen.

Metalloxidbeschichtete silikatische Pigmente sind allgemein bekannt und auch unter den Bezeichnungen Iriodin® (Merck, Darmstadt), Flonac® (Kemira Oy, Pori) oder Mearlin® (Mearl Corporation, New York) im Handel.

Durch geeignete Wahl der silikatischen Pigmente (II) kann das Farbenspiel der Metallpigmente (I) variiert oder ergänzt werden.

Zeigt beispielsweise ein mit (A) und (B) beschichtetes metallisches Substrat bei einem Betrachtungswinkel von 25° einen goldenen Farbton, so kann dieser durch Beimischen eines rotgoldene Interferenzfarbe aufweisenden titandioxidbeschichteten Glimmerpigments zu dem nur mit (A) beschichteten Metallpigment und anschließende gemeinsame Beschichtung mit (B) in Richtung auf einen rötlicheren Farbton verschoben werden.

Die Zusammensetzung der erfindungsgemäßen Glanzpigmentmischungen ist nicht kritisch und wird von den gewünschten Koloristik bestimmt.

Prinzipiell kann das Gewichtsverhältnis metallisches Pigment (I): silikatisches Pigment (II) von 1:99 bis 99:1 variiert werden. Um ein ausreichendes Deckvermögen zu erreichen, enthalten die erfindungsgemäßen Pigmentmischungen vorzugsweise mindestens 5 Gew.-% metallisches Glanzpigment (I).

Der bevorzugte Weg zur Herstellung der erfindungsgemäßen Pigmentmischungen ist die gemeinsame Beschichtung der bereits gemäß Schritt (a) mit der Schicht (A) und mit einer Schicht (A') belegten Substratteilchen mit der schwarzen Schicht (B) und gewünschtenfalls der Deckschicht (C).

Selbstverständlich können jedoch auch alle Schichten getrennt aufgebracht werden und die beschichteten Pigmente dann anschließend gemischt werden. Bei dieser Vorgehensweise können die Schichten (B) und (B') sowie (C) und (C') zusätzlich variiert werden.

Bei dem erfindungsgemäßen Verfahren zur Herstellung der mehrfach beschichteten Glanzpigmente wird die Schicht (A) naßchemisch durch Hydrolyse organischer Silicium- und/oder Aluminiumverbindungen, bei denen die organischen Reste über Sauerstoffatome an die Metalle gebunden sind, in Gegenwart eines organischen Lösungsmittels aufgebracht.

Als organische Lösungsmittel eignen sich hierfür sowohl aprotische Lösungsmittel wie Ketone, β-Diketone, Ether, vor allem cyclische Ether, und stickstoffhaltige Lösungsmittel, z.B. auch amidische Lösungsmittel, als auch protische Lösungsmittel wie ein- oder mehrwertige Alkohole mit vorzugsweise 1 bis 6 Kohlenstoffatomen, die mit Wasser mischbar sind.

Beispiele für bevorzugte Lösungsmittel sind Aceton, Tetrahydrofuran, Ethanol und n- und iso-Propanol sowie Diethylketon, Acetylaceton, Dioxan, Trioxan, Ethylenglykol, Propylenglykol, Glycerin, Dimethylformamid, Dimethylacetamid, N-Methylpyrrolidon, Pyridin und Acetonitril.

Als metallische Ausgangsverbindungen sind in den genannten organischen Lösungsmitteln lösliche organische Verbindungen, bei denen die organischen Reste über Sauerstoffatome an die Metalle gebunden sind, geeignet. Bevorzugte Beispiele sind die Acetylacetonate und insbesondere Alkoholate, vor allem C₁-C₄-Alkanolate, z.B. Aluminiumtriisopropanolat und Tetraethoxysilan.

Die Hydrolyse wird vorzugsweise in Gegenwart einer Base oder einer Säure als Katalysator durchgeführt. Hierfür eignen sich z.B. neben Alkalilaugen wie Natronlauge insbesondere wäßrige Ammoniaklösungen. Geeignete saure Katalysatoren sind beispielsweise Phosphorsäure und organische Säuren wie Essigsäure und Oxalsäure.

Wasser muß mindestens in der stöchiometrisch für die Hydrolyse erforderlichen Menge vorliegen, bevorzugt ist jedoch die 2 bis 100fache, insbesondere die 5 bis 20fache Menge.

Bezogen auf die eingesetzte Wassermenge, gibt man in der Regel 3 bis 40 Vol.-%, vorzugsweise 5 bis 30 Vol.-%, einer 25 gew.-%igen wäßrigen Ammoniaklösung zu.

Für die Temperaturführung hat es sich als vorteilhaft erwiesen, das Reaktionsgemisch innerhalb von 10 bis 48 h schrittweise auf Rückflußtemperatur zu erhitzen. Bei Verwendung von iso-Propanol als Lösungsmittel rührt man das Gemisch zum Beispiel bevorzugt zunächst 4 bis 20 h bei 40°C, dann 4 bis 20 h bei 60°C und zum Schluß 2 bis 8 h bei 80°C.

Verfahrenstechnisch geht man bei Schritt a) des erfindungsgemäßen Herstellungsverfahrens zweckmäßigerweise wie folgt vor:

Man legt Substratteilchen, organisches Lösungsmittel, Wasser und Katalysator (Base oder Säure) vor und gibt die zu hydrolysierende Metallverbindung, pur oder gelöst, z.B. als 30 bis 70, bevorzugt 40 bis 60 vol.-%ige Lösung im organischen Lösungsmittel, zu. Erfolgt die Zugabe der Metallverbindung in einem Schritt, dann wird die Suspension anschließend wie oben beschrieben unter Rühren erhitzt. Man kann die Metallverbindung aber auch bei erhöhter Temperatur kontinuierlich zudosieren, wobei das Wasser vorzugsweise nicht vorgelegt wird, sondern ebenfalls kontinuierlich zudosiert wird. Nach beendeter Beschichtung wird die Reaktionsmischung wieder auf Raumtemperatur abgekühlt.

Um eine Agglomeratbildung während des Beschichtungsvorgangs zu verhindern, kann die Suspension einer starken mechanischen Beanspruchung wie Pumpen, kräftigem Rühren oder Einwirken von Ultraschall unterzogen werden.

Gewünschtenfalls kann man den Beschichtungsschritt ein- oder mehrfach wiederholen. Sollte die Mutterlauge milchig trüb aussehen, so empfiehlt es sich, diese vor einer weiteren Beschichtung auszutauschen.

Die Isolierung der mit der Schicht (A) belegten Substratteilchen kann in einfacher Weise durch Abfiltrieren, Waschen mit organischem Lösungsmittel, vorzugsweise den auch als Lösungsmittel verwendeten Alkoholen, und anschließendes Trocknen (üblicherweise 2 bis 24 h bei 20 bis 200°C) erfolgen.

Mit Hilfe des erfindungsgemäßen Verfahrens können auch dickere Siliciumoxidschichten von z.B. ≥ 70 nm problemlos in guter Qualität, d.h. als zusammenhängender, zur Interferenz befähigter Film aufgebracht werden.

Je nach der Teilchengröße der verwendeten Substratteilchen werden in der Regel 10 bis 60 Gew.-% Metalloxid (A), bezogen auf das beschichtete Substrat, zur Erzielung der gewünschten Farbeffekte eingesetzt. So sind beispielsweise bei gröberen Aluminiumteilchen (um 1,5 m²/g) bereits bei etwa 15 Gew.-% Siliciumoxid und bei feineren Aluminiumteilchen (um 4,5 m²/g) ab etwa 30 Gew.-% Siliciumoxid attraktive Farbeffekte zu beobachten.

Die weiteren Schichten (B) und (C) werden bei dem erfindungsgemäßen Verfahren vorzugsweise wie in der EP-A-571 836 beschrieben über Gasphasenzersetzung flüchtiger Metallverbindungen (chemical vapor deposition, CVD) auf die mit (A) belegten Substratteilchen aufgebracht.

Hierfür wird zweckmäßigerweise ein beheizbarer Wirbelschichtreaktor, wie er beispielsweise in der EP-A-33 457 oder der DE-A-38 13 335 beschrieben ist, verwendet, in dem die mit (A) beschichteten Substratteilchen zunächst mit einem Wirbelgas fluidisiert und auf die für die Zersetzung der jeweiligen Metallverbindung erforderliche Temperatur von in der Regel 70 bis 350°C erhitzt werden. Die in einem vorgeschalteten Verdampfergefäß unter Verwendung eines geeigneten Trägergases verdampften Metallverbindungen sowie die gegebenenfalls zur Zersetzung benötigten Gase werden dann über getrennte Düsen eingetragen.

Metallische Schichten (B) werden dabei bevorzugt durch inerte Zersetzung von Metallcarbonylen, wie Eisenpentacarbonyl, Chrom-, Molybdän-, Wolframhexacarbonyl, Nickeltetracarbonyl und Dicobaltoctacarbonyl, aufgebracht (Schritt (b1), wobei z.B. bei der Zersetzung von Mo(CO)₆ Temperaturen von 200 bis 250°C bevorzugt sind.

Gemischte Metallschichten (B), die z.B. im wesentlichen aus Molybdän und Chrom bestehen, können durch gleichzeitige oder durch aufeinanderfolgende Gasphasenbeschichtung aufgebracht werden. Die zweite Variante bietet sich insbesondere bei dünnen Schichten (B) an, da eine Durchmischung der abgeschiedenen Schichten erfolgt.

Soll die zweite, schwarze Schicht aus niederen Metalloxiden, wie Magnetit, VO₂ oder V₂O₃, bestehen (Schritt (b3)), dann werden zweckmäßigerweise die Metallcarbonyle wie Eisenpentacarbonyl oder Oxychloride wie Vanadinoxychlorid mit Wasserdampf zersetzt. Werden bei dieser Gasphasenzersetzung zunächst höhere Metalloxide, beispielsweise V₂O₅, abgeschieden, so müssen diese anschließend z.B. mit Wasserstoff oder Ammoniak zum gewünschten Oxid reduziert werden.

Die Schicht (B) wird nach beendeter Beschichtung, insbesondere wenn sie die äußere Schicht des Glanzpigmentes bilden soll, zweckmäßigerweise an der Oberfläche passiviert. Das kann in einfacher Weise geschehen, indem den Wirbelgasen bei der Abkühlung etwas Luft zugemischt wird.

Zur Abscheidung der farblosen oder selektiv absorbierenden Metalloxidschicht (C) werden in Schritt (cl) vorzugsweise Metallcarbonyle, wie Eisenpentacarbonyl und Chromhexacarbonyl, mit Sauerstoff (bzw. Luft) zersetzt, Metallhalogenide, wie Silicium-, Titan- und Zirkontetrachlorid, und Metallalkoholate, wie Titan- und Zirkontetra-n- und iso-propanolat, mit Wasserdampf zersetzt.

Weitere Einzelheiten zu der CVD-Verfahrensvariante sind in der EP-A-571 836 beschrieben.

Metallische Schichten (B) können aber auch naßchemisch durch Reduktion aus geeigneten Metallsalzlösungen aufgebracht werden (Schritt (b2)). Auf diese Weise können vor allem edlere Metalle wie Kupfer, Silber, Gold, Cobalt, Nickel, Palladium und Platin abgeschieden werden. Wie in der EP-A-353 544 beschrieben, eignen sich hierfür eine Reihe von Reduktionsmitteln, insbesondere milde organische Reduktionsmittel wie Glucose und Formaldehyd.

Zum Aufbau der metallischen Schichten (B) eignen sich auch Metallegierungen wie NiP, NiB, NiCo, NiWP, CoP und AgAu, die ebenfalls naßchemisch (z.B. durch Reaktion einer Metallsalzlösung mit Hypophospit) aufzubringen sind (EP-A-313 281).

In der Regel werden jedoch die über die Gasphase aufgebrachten Metallschichten aufgrund ihrer höheren Qualität (feiner kristallin, filmartig) den naßchemisch aufgebrachten vorzuziehen sein, da sie meist brillantere und farbstärkere Glanzpigmente ergeben.

Schließlich kann auch die Metalloxidschicht (C), wie oben für die Schicht (A) beschrieben, naßchemisch aufgebracht werden (Schritt (c2)). Diese Vorgehensweise ist insbesondere dann geeignet, wenn die Schicht (C) im wesentlichen aus Silicium- und/ oder Aluminiumoxid bestehen soll.

In Abhängigkeit von der Vollständigkeit der an den Schritt (a) oder (c2) anschließenden Trocknung können diese Metalloxidschichten noch geringe Mengen Wasser enthalten, die Metalloxide also teilweise als Oxidhydrate vorliegen.

Mit Hilfe des erfindungsgemäßen Herstellungsverfahrens können die mehrfach beschichteten metallischen Glanzpigmente in einfacher Weise in großen Mengen reproduzierbar hergestellt werden. Es werden vollständig umhüllte Pigmentteilchen mit hoher Qualität der einzelnen Beschichtungen erhalten.

Gewünschtenfalls können die beschichteten Glanzpigmente zur Desagglomerierung und Glättung einem zusätzlichen Veredelungsschritt durch schonendes Aufmahlen in einer Kugelmühle oder vergleichbaren Apparaten unterzogen werden.

Die erfindungsgemäßen Glanzpigmente und ihre Mischungen mit silikatischen Pigmenten eignen sich vorteilhaft für viele Zwecke, wie zur Einfärbung von Kunststoffen, Gläsern, keramischen Produkten, Zubereitungen der dekorativen Kosmetik und insbesondere von Tinten, Lacken und Druckfarben, vor allem Sicherheitsdruckfarben. Bei der Applikation im Druck sind alle industrieüblichen Druckverfahren, z.B. Siebdruck, Tiefdruck, Bronzierdruck, Flexodruck und Offsetdruck, geeignet.

Weiterhin lassen sich die erfindungsgemäßen Pigmente auch vorteilhaft in Abmischung mit transparenten und deckenden Weiß-, Bunt- und Schwarzpigmenten sowie auch herkömmlichen Glanzpigmenten auf der Basis von metalloxidbeschichteten Glimmer- und Metallpigmenten und plättchenförmigen Eisenoxiden verwenden.

### Beispiele

### Herstellung und Anwendung von erfindungsgemäßen Glanzpigmenten

Bei der Einarbeitung der Pigmente in Lack wurden jeweils 0,4 g Pigment in 3,6 g eines Polyester-Mischlackes mit 21 Gew.-% Feststoffanteil eingerührt und 2 min im Red Devil dispergiert. Mit einer Rakel (160 µm Naßfilmdicke) wurden auf schwarzweißem Karton Abzüge der pigmentierten Lacke angefertigt.

Bei der Anwendung der Pigmente im Bronzierdruck wurden Papierbögen zunächst im Offset-Verfahren mit einem nichtpigmentierten Klebefirnis (Bronzierfirnis) aus 95 Gew.-% Leinölfirnis und phenolmodifiziertem Kolophoniumharzester und 5 Gew.-% Polyvinyltoluol bedruckt und dann sofort in die Bronzierstation weitergeführt, wo sie mit dem Pigment bestäubt wurden. Überschüssiges Pigmentpulver wurde anschließend mit einer Samtrakel entfernt.

Bei der Anwendung der Pigmente im Siebdruck wurden 10 g Pigment in 90 g einer handelsüblichen Bindemittellösung (22,5 g PVC-Mischpolymerisat Laroflex® MP45, 4,5 g Methoxypropylacetat, 13,5 g n-Hexyldiglykol, 49,5 g Butylglykol) eingerührt. Die so hergestellte Siebdruckfarbe wurde mit einer handelsüblichen Siebdruckmaschine (Siebmaschenweite 112 bis 150 µm) auf gestrichenes, titandioxidbeschichtetes Papier in einer Schichtdicke von 45 µm aufgebracht und an der Luft getrocknet.

### Beispiel 1

a) In einem mit Rückflußkühler und Rührapparatur versehenen Rundkolben wurden 150 g Aluminiumpulver (BET-Oberfläche 1,5 m²/g, mitlerer Teilchendurchmesser 60 µm) in 750 ml Isopropanol aufgeschlämmt. Nach Zugabe einer Lösung aus 1125 ml Isopropanol, 103,3 g Wasser und 28,3 g einer 25 gew.-%igen wäßrigen Ammoniaklösung wurde die Suspension unter Rühren auf 40°C erwärmt. Anschließend wurde die Reaktionsmischung nach Zugabe von 136,3 g Tetraethoxysilan 4 h bei 40°C, 1 h bei 60°C und 1 h bei 80°C gerührt.
   Nach Abkühlen der Suspension wurde das nach wie vor silbrig glänzende Produkt von der Mutterlauge abfiltriert, mit Isopropanol gewaschen und getrocknet.
   Das beschichtete Aluminiumpulver hatte einen SiO₂-Gehalt von 13,5 Gew.-% und sah unverändert aus.
b) Anschließend wurde das beschichtete Aluminiumpulver im Wirbelschichtreaktor (beschrieben in der EP-A-571 836) unter Fluidisierung mit insgesamt 700 l/h Stickstoff auf 220°C erhitzt. Aus einer auf 70°C erwärmten Vorlage wurden mit einem Stickstoffstrom von 400 l/h 61 g Molybdänhexacarbonyl in 8 h in den Reaktor überführt, wo sie zu Molybdän und Kohlenmonoxid zersetzt wurden.
   Nach beendeter Molybdänabscheidung wurde den Wirbelgasen zur Passivierung der Molybdänoberfläche etwas Luft zugesetzt.

Das erhaltene Pigment hatte einen Molybdängehalt von 5 Gew.-% und zeigte, im Lack appliziert, bei nahezu unverändert starkem metallischen Glanz eine kräftige, grünstichig blaue Interferenzfarbe, die bei steileren Betrachtungswinkeln in ein rotstichiges Blau abkippte. Nach rasterelektronenmikroskopischen Untersuchungen ergab sich eine filmartige Abscheidung des Molybdäns.

### Beispiel 2

a) Das Aluminiumpulver wurde analog zu Beispiel 1a) zweimal mit SiO₂ beschichtet. Das beschichtete Aluminium hatte einen SiO₂-Gehalt von 18,8 Gew.-% und zeigte einen schwach goldenen Schimmer.
b) Analog zu Beispiel 1b) wurde das beschichtete Aluminiumpulver dann unter Fluidisierung mit insgesamt 800 l/h Stickstoff bei 200°C in 3,5 h unter Verwendung von 18,5 g Mo(CO)₆ filmartig mit Molybdän beschichtet.

Das erhaltene Pigment hatte einen Molybdängehalt von 2,2 Gew.-% und zeigte, im Lack appliziert, bei nahezu unverändert starkem metallischen Glanz eine kräftige, rötlich goldene Interferenzfarbe, die bei steileren Betrachtungswinkeln ins grünlich Goldene abkippte.

### Beispiel 3

a) Das Aluminiumpulver wurde analog zu Beispiel 1a) dreimal mit SiO₂ beschichtet. Das beschichtete Aluminium hatte einen SiO₂-Gehalt von 29,9 Gew.-% und zeigte einen schwach rötlichen Schimmer.
b) Analog zu Beispiel 1 b) wurde das beschichtete Aluminiumpulver dann in 6 h unter Verwendung von 30 g Mo(CO)₆ filmartig mit Molybdän beschichtet.

Das erhaltene Pigment hatte einen Molybdängehalt von 3,0 Gew.-% und zeigte, im Lack appliziert, eine kräftige, rote Interferenzfarbe, die bei steileren Betrachtungswinkeln über gold nach grün abkippte.

### Beispiel 4

a) Das Aluminiumpulver wurde analog zu Beispiel 1a) dreimal mit SiO₂ beschichtet, im 3. Beschichtungszyklus wurden jedoch 150,0 g Tetraethoxysilan eingesetzt. Das beschichtete Aluminium hatte einen SiO₂-Gehalt von 31,0 Gew.-% und zeigte einen schwach rötlichen Schimmer.
b) Analog zu Beispiel 1 b) wurde das beschichtete Aluminiumpulver dann in 8 h unter Verwendung von 33 g Mo(CO)₆ filmartig mit Molybdän beschichtet.

Das erhaltene Pigment hatte einen Molybdängehalt von 3,8 Gew.-%, war intensiv lila gefärbt und zeigte, im Siebdruck appliziert, bei steileren Betrachtungswinkeln einen Farbflop nach grünlich gold.

### Beispiel 5

a) Eine Aufschlämmung von 150 g des Aluminiumpulvers aus Beispiel 1 in 1500 ml Isopropanol wurde nach Zugabe einer Lösung aus 500 ml Isopropanol, 100 ml Wasser und 30 g einer 25 gew.-%igen wäßrigen Ammoniaklösung unter Rühren auf 40°C erwärmt. Dann wurden zunächst in 1,5 h 136 g Tetraethoxysilan und anschließend in 4,5 h kontinuierlich weitere 408 g Tetraethoxysilan sowie parallel dazu weitere 75 ml Wasser zugegeben. Danach wurde die Mischung 12 h bei 60°C und 3 h bei 80°C gerührt.
   Das analog Beispiel la) isolierte beschichtete Aluminiumpulver hatte einen SiO₂-Gehalt von 20,8 Gew.-% und zeigte einen sehr schwach goldenen Schimmer.
b) Analog zu Beispiel 1 b) wurde das beschichtete Aluminiumpulver dann in 5 h unter Verwendung von 25,5 g Mo(CO)6 filmartig mit Molybdän beschichtet.

Das erhaltene Pigment hatte einen Molybdängehalt von 1,3 Gew.-%, war intensiv rotgold gefärbt und zeigte, im Bronzierdruck appliziert, bei steileren Betrachtungswinkeln einen Farbflop nach grünlich gold.

### Beispiel 6

Zu einer vor Licht geschützten Suspension von 10 g des in Beispiel 5 SiO₂-beschichteten Aluminiumpulvers in 150 ml Wasser wurden 1,8 g Silbernitrat, gelöst in 40 ml Wasser und 3,6 ml einer 25 gew.-%igen wäßrigen Ammoniaklösung, und 20 ml einer 75 gew.-%igen Natronlauge gegeben. Dann wurde in 5 h eine Lösung von 2,4 g Glucose in 150 ml Wasser zugetropft.

Nach einstündigem Rühren bei Raumtemperatur wurde das mit Silber beschichtete Aluminiumpulver abfiltriert, mit Wasser gewaschen und bei 60°C getrocknet.

Das erhaltene Pigment hatte einen Silbergehalt von 9,1 Gew.-%, war schwach rot gefärbt und zeigte, im Lack appliziert, bei steileren Betrachtungswinkeln einen Farbflop nach grünlich gold.

### Beispiel 7

a) Eine Aufschlämmung von 150 g des Aluminiumpulvers aus Beispiel 1 in 750 ml Isopropanol wurde nach Zugabe einer Lösung aus 750 ml Isopropanol, 100 ml Wasser und 28,3 g einer 25 gew.-%igen wäßrigen Ammoniaklösung unter Rühren auf 40°C erwärmt. Dann wurden zunächst in 2 h 136 g Tetraethoxysilan, gelöst in 125 ml Isopropanol, und anschließend in 5 h weitere 272 g Tetraethoxysilan, gelöst in 250 ml Isopropanol, sowie parallel dazu weitere 48 ml Wasser zugegeben. Danach wurde die Mischung 12 h bei 40°C gerührt, in 6 h kontinuierlich auf 80°C erhitzt und 3 h bei dieser Temperatur gerührt.
   Das analog Beispiel 1a) isolierte beschichtete Aluminiumpulver hatte einen SiO₂-Gehalt von 25,7 Gew.-% und zeigte einen sehr schwach grünlichen Farbschimmer.
b) Anschließend wurde das beschichtete Aluminiumpulver im Wirbelschichtreaktor unter Fluidisierung mit insgesamt 800 l/h Stickstoff auf 200°C erhitzt. Aus einer auf 20°C temperierten Vorlage wurden mit einem Stickstoffstrom von 400 l/h 26,1 g Eisenpentacarbonyl in 4 h in den Reaktor überführt, wo sie zu Eisen und Kohlenmonoxid zersetzt wurden.
   Nach beendeter Eisenabscheidung wurde den Wirbelgasen zur Passivierung der Eisenoberfläche etwas Luft zugesetzt.

Das erhaltene Pigment hatte einen Eisengehalt von 3,0 Gew.-%, war grünlich gold gefärbt und zeigte, im Lack appliziert, bei steileren Betrachtungswinkeln einen Farbflop nach blau.

### Beispiel 8

a) Das Aluminiumpulver wurde analog zu Beispiel 1a) zweimal mit SiO₂ beschichtet, im 2. Beschichtungszyklus wurden jedoch 150,0 g Tetraethoxysilan eingesetzt. Das beschichtete Aluminium hatte einen SiO₂-Gehalt von 24,3 Gew.-% und zeigte einen schwach violetten Schimmer.
b) Anschließend wurde das beschichtete Aluminiumpulver analog zu Beispiel 1a) unter Verwendung von 30 g Chromhexacarbonyl (Verdampfertemperatur 80°C) in 8 h bei 240°C zunächst mit Chrom beschichtet, wobei ein stärker violett gefärbtes Pigment mit einem Chromgehalt von 1,7 Gew.-% erhalten wurde.
   Dann wurde das Pigment unter Verwendung von 19,4 g Molybdänhexacarbonyl (Verdampfertemperatur 70°C) in 4 h bei 210°C noch zusätzlich mit Molybdän beschichtet.

Das erhaltene Pigment hatte einen Molybdängehalt von 1,1 Gew.-% und zeigte, im Lacke appliziert, eine kräftige, blaue Interferenzfarbe, die bei steileren Betrachtungswinkeln nach Violett abkippte.

### Beispiel 9

a) 150 g feinteiliges Aluminiumpulver (BET-Oberfläche 4,5 m²/g, mittlerer Teilchendurchmesser 20 µm) wurden analog zu Beispiel 1a) sechsmal mit SiO₂ beschichtet. Das beschichtete Aluminium hatte einen SiO2-Gehalt von 45,0 Gew.-% und zeigte einen sehr schwach goldenen Schimmer.
b) Analog zu Beispiel 1b), jedoch unter Fluidisierung mit insgesamt 1300 l/h Stickstoff, wurde das beschichtete Aluminiumpulver dann in 8 h unter Verwendung von 40,0 g Mo(CO)₆ mit Molybdän beschichtet.

Das erhaltene Pigment hatte einen Molybdängehalt von 6,1 Gew.-% und zeigte, im Lack appliziert, bei starkem metallischen Glanz eine kräftige, rotgoldene Interferenzfarbe, die bei steileren Betrachtungswinkeln ins Grüngoldene abkippte.

### Beispiel 10

a) Eine Aufschlämmung von 200 g des Aluminiumpulvers aus Beispiel 1 in 1500 ml Isopropanol wurde nach Zugabe einer Lösung aus 500 ml Wasser und 40 ml einer 25 gew.-%igen wäßrigen Ammoniaklösung unter Rühren auf 60°C erwärmt. Gleichzeitig wurde begonnen, ein Gemisch aus 600 ml Isopropanol und 600 g Tetraethoxysilan mit einer Dosiergeschwindigkeit von 100 ml/h zuzugeben. Nach 12 h war die gesamte Menge zudosiert. Nach weiterem 12stündigen Nachrühren wurde analog Beispiel la) aufgearbeitet.
   Das beschichtete Aluminiumpulver hatte einen SiO2-Gehalt von 24,6 Gew.-% und zeigte einen schwach rötlichen Schimmer.
b) Anschließend wurden 200 g des beschichteten Aluminiumpulvers im Wirbelschichtreaktor unter Fluidisierung mit insgesamt 1200 l/h Stickstoff auf 300°C erhitzt. Aus einer auf 80°C erwärmten Vorlage wurden mit einem Teil der Wirbelgase (400 l/h) 48,7 g Chromhexacarbonyl in 14 h in den Reaktor überführt, wo sie zu Chrom und Kohlenmonoxid zersetzt wurden. Nach beendeter Chromabscheidung wurde den Wirbelgasen zur Passivierung der Chromoberfläche etwas Luft zugesetzt.

Das erhaltene Pigment hatte einen Chromgehalt von 3,9 Gew.-% und zeigte, im Lack appliziert, bei starkem metallischen Glanz eine rotgoldene Interferenzfarbe, die bei steileren Betrachtungswinkeln nach Grün abkippte.

### Beispiel 11

a) Eine Aufschlämmung von 100 g des Aluminiumpulvers aus Beispiel 9 in 3000 ml Isopropanol wurde nach Zugabe einer Lösung aus 1000 ml Wasser und 80 ml einer 25 gew.-%igen wäßrigen Ammoniaklösung unter Rühren auf 60°C erwärmt, wobei gleichzeitig mit der Zudosierung eines Gemisches aus 1100 ml Iso-propanol und 1100 g Tetraethoxysilan (Dosiergeschwindigkeit 100 ml/h) begonnen wurde. Nach 22 h war die gesamte Menge zudosiert. Nach weiterem 20stündigen Nachrühren wurde analog Beispiel 1a) aufgearbeitet.
   Das beschichtete Aluminiumpulver hatte einen SiO2-Gehalt von 73,6 Gew.% und zeigte einen schwachen Grün/Rot-Flop.
b) Anschließend wurden 140 g des beschichteten Aluminiumpulvers im Wirbelschichtreaktor unter Fluidisierung mit insgesamt 1000 l/h Stickstcff aus 220°C erhitzt. Aus einer auf 70°C erwärmten Vorlage wurden analog Beispiel 10b) mit einem Teil der Wirbelgase (400 l/h) 18,0 g Chromhexacarbonyl in 6 h in den Reaktor überführt.

Das erhaltene Pigment hatte einen Chromgehalt von 2,5 Gew.-% und zeigte, im Lack appliziert, bei starkem metallischen Glanz eine grüne Interferenzfarbe, die bei steileren Betrachtungswinkeln nach Rot abkippte.

### Beispiel 12

a) Analog Beispiel lla) wurde das Aluminiumpulver mit SiO2 beschichtet.
b) Anschließend wurden 140 g des beschichteten Aluminiumpulvers analog zu Beispiel 12b) in 8 h Verwendung von 20,0 g Wolframhexacarbonyl (Temperatur der Vorlage: 80°C) beschichtet.

Das erhaltene Pigment hatte einen Wolframgehalt von 5,5 Gew.-% und zeigte, im Lack appliziert, bei starkem metallischen Glanz eine grüne Interferenzfarbe, die bei steileren Betrachtungswinkeln nach Rot abkippte.

## Patentansprüche

1. Glanzpigmente auf der Basis von mehrfach beschichteten plättchenförmigen metallischen Substraten mit
A) einer ersten, im wesentlichen aus Siliciumoxid, Siliciumoxidhydrat, Aluminiumoxid und/oder Aluminiumoxidhydrat bestehenden Schicht,
B) einer zweiten, im wesentlichen aus Metall und/oder nichtselektiv absorbierendem Metalloxid bestehenden Schicht und
C) gewünschtenfalls einer dritten, im wesentlichen aus farblosem oder selektiv absorbierendem Metalloxid bestehenden Schicht,
erhältlich durch
a) naßchemische Belegung der Substratteilchen mit Siliciumoxid, Siliciumoxidhydrat, Aluminiumoxid und/oder Aluminiumoxidhydrat durch hydrolytische Zersetzung von organischen Silicium- und/oder Aluminiumverbindungen, bei denen die organischen Reste über Sauerstoffatome an die Metalle gebunden sind, in Gegenwart eines organischen Lösungsmittels, in welchem die Metallverbindungen löslich sind, und gegebenenfalls anschließende Trocknung,
b) weitere Belegung der in Schritt a) erhaltenen Teilchen mit Metall durch
b1) Gasphasenzersetzung flüchtiger Metallverbindungen in einer inerten Atmosphäre oder
b2) stromlose, naßchemische Metallabscheidung und gegebenenfalls anschließende Trocknung
oder mit nichtselektiv absorbierendem Metalloxid durch
b3) Gasphasenzersetzung flüchtiger Metallverbindungen in Gegenwart von Sauerstoff und/oder Wasserdampf und
c) gewünschtenfalls zusätzliche Belegung der in Schritt b) erhaltenen Teilchen mit farblosem oder selektiv absorbierendem Metalloxid durch
c1) Gasphasenzersetzung flüchtiger Metallverbindungen in Gegenwart von Sauerstoff und/oder Wasserdampf oder
c2) naßchemische Belegung durch hydrolytische Zersetzung organischer Metallverbindungen, bei denen die organischen Reste über Sauerstoffatome an die Metalle gebunden sind, in Gegenwart eines organischen Lösungsmittels, in welchem die Metallverbindungen löslich sind, und anschließende Trocknung.

2. Glanzpigmente nach Anspruch 1, bei denen die Schicht (B) im wesentlichen aus Chrom, Molybdän, Wolfram, Eisen, Cobalt, Nickel, Silber, Kupfer, Gold, Palladium, Platin, Nickel-, Cobalt-, Silber/Gold-Legierungen, Magnetit, Nickeloxid, Cobaltoxid und/oder Vanadiumoxid besteht.

3. Glanzpigmente nach Anspruch 1 oder 2, bei denen die Schicht (C) im wesentlichen aus Siliciumoxid, Siliciumoxidhydrat, Aluminiumoxid, Aluminiumoxidhydrat, Titanoxid, Zirkonoxid, Chromoxid und/oder Eisenoxid besteht.

4. Glanzpigmente nach den Ansprüchen 1 bis 3, bei denen das metallische Substrat im wesentlichen aus Aluminiumplättchen, die passiviert sein können, besteht.

5. Glanzpigmente auf der Basis von mehrfach beschichteten, im wesentlichen aus Aluminium bestehenden, plättchenförmigen Substraten mit
A) einer ersten, 50 bis 600 nm dicken, im wesentlichen aus Siliciumoxid und/oder Siliciumoxidhydrat bestehenden Schicht,
B) einer zweiten, 1 bis 25 nm dicken, im wesentlichen aus Molybdän, Chrom, Wolfram und/oder Eisen bestehenden Schicht und
C) gewünschtenfalls einer dritten, 5 bis 250 nm dicken, im wesentlichen aus Siliciumoxid, Siliciumoxidhydrat, Aluminiumoxid und/oder Aluminiumoxidhyrat bestehenden Schicht.

6. Glanzpigmentmischungen aus
I) den Glanzpigmenten gemäß den Ansprüchen 1 bis 5 und
II) mehrfach beschichteten silikatischen Plättchen, die
A') eine erste, im wesentlichen aus farblosem oder selektiv absorbierendem Metalloxid bestehende Schicht,
B') eine zweite, im wesentlichen aus Metall und/oder nichtselektiv absorbierendem Metalloxid bestehende Schicht und
C') gewünschtenfalls eine dritte, im wesentlichen aus farblosem oder selektiv absorbierendem Metalloxid bestehende Schicht
aufweisen, als wesentlichen Komponenten.

7. Verfahren zur Herstellung von Glanzpigmenten auf der Basis von mehrfach beschichteten plättchenförmigen metallischen Substraten, dadurch gekennzeichnet, daß man es gemäß den in Anspruch 1 definierten Verfahrensschritten vornimmt.

8. Verwendung von Glanzpigmenten gemäß den Ansprüchen 1 bis 6 zur Einfärbung von Lacken, Druckfarben, Tinten, Kunststoffen, Gläsern, keramischen Produkten und Zubereitungen der dekorativen Kosmetik.

## Claims

1. Luster pigments based on multiply coated plateletlike metallic substrates comprising
A) a first layer consisting essentially of silicon oxide, silicon oxide hydrate, aluminum oxide and/or aluminum oxide hydrate,
B) a second layer consisting essentially of metal and/or nonselectively absorbing metal oxide, and
C) if desired, a third layer consisting essentially of colorless or selectively absorbing metal oxide,
obtainable by
a) wet-chemical coating of the substrate particles with silicon oxide, silicon oxide hydrate, aluminum oxide and/ or aluminum oxide hydrate by hydrolytic decomposition of organic silicon and/or aluminum compounds in which the organic radicals are attached to the metals via oxygen atoms in the presence of an organic solvent in which the metal compounds are soluble with or without subsequent drying,
b) further coating of the particles obtained in step a) with metal by
b1) gas phase decomposition of volatile metal compounds in an inert atmosphere or
b2) electroless, wet-chemical metal deposition with or without subsequent drying
or with nonselectively absorbing metal oxide by
b3) gas phase decomposition of volatile metal compounds in the presence of oxygen and/or water vapor and
c) if desired additional coating of the particles obtained in step b) with colorless or selectively absorbing metal oxide by
c1) gas phase decomposition of volatile metal compounds in the presence of oxygen and/or water vapor or
c2) wet-chemical coating by hydrolytic decomposition of organic metal compounds in which the organic radicals are attached to the metals via oxygen atoms in the presence of an organic solvent in which the metal compounds are soluble and subsequent drying.

2. Luster pigments as claimed in claim 1 wherein the layer (B) consists essentially of chromium, molybdenum, tungsten, iron, cobalt, nickel, silver, copper, gold, palladium, platinum, nickel alloys, cobalt alloys, silver-gold alloys, magnetite, nickel oxide, cobalt oxide and/or vanadium oxide.

3. Luster pigments as claimed in claim 1 or 2 wherein the layer (C) consists essentially of silicon oxide, silicon oxide hydrate, aluminum oxide, aluminum oxide hydrate, titanium oxide, zirconium oxide, chromium oxide and/or iron oxide.

4. Luster pigments according to any of claims 1 to 3 wherein the metallic substrate consists essentially of passivated or non-passivated aluminum platelets.

5. Luster pigments based on multiply coated plateletlike substrates consisting essentially of aluminum comprising
A) a first layer from 50 to 600 nm in thickness consisting essentially of silicon oxide and/or silicon oxide hydrate,
B) a second layer from 1 to 25 in thickness consisting essentially of molybdenum, chromium, tungsten and/or iron, and
C) if desired, a third layer from 5 to 250 nm in thickness consisting essentially of silicon oxide, silicon oxide hydrate, aluminum oxide and/or aluminum oxide hydrate.

6. Luster pigment mixtures of
I) the luster pigments of any of claims 1 to 5 and
II) multiply coated silicatic platelets comprising
A') a first layer consisting essentially of colorless or selectively absorbing metal oxide,
B') a second layer consisting essentially of metal and/or nonselectively absorbing metal oxide, and
C') if desired, a third layer consisting essentially of colorless or selectively absorbing metal oxide,
as essential components.

7. A process for producing luster pigments based on multiply coated plateletlike metallic substrates, which comprises the process steps defined in claim 1.

8. The use of luster pigments as claimed in any of claims 1 to 6 for coloring coatings, inks, including printing inks, plastics, glasses, ceramic products and decorative cosmetic preparations.

## Revendications

1. Pigments brillants à base de substrats métalliques stratifiés sous forme de plaquettes à revêtements multiples avec
A) une première couche constituée essentiellement d'oxyde de silicium, d'oxyde de silicium hydraté, d'oxyde d'aluminium et/ou d'oxyde d'aluminium hydraté,
B) une deuxième couche constituée essentiellement de métal et/ou d'oxyde métallique n'absorbant pas de façon sélective et
C) une éventuelle troisième couche constituée essentiellement d'oxyde métallique incolore ou absorbant de façon sélective,
pouvant être obtenus par
a) un recouvrement par voie chimique humide des particules de substrats avec de l'oxyde de silicium, de l'oxyde de silicium hydraté, de l'oxyde d'aluminium et/ou de l'oxyde d'aluminium hydraté, par décomposition par hydrolyse de composés organiques du silicium et/ou de l'aluminium dans lesquels les restes organiques sont reliés aux métaux par des atomes d'oxygène, en présence d'un solvant organique dans lequel les composés métalliques sont solubles, suivi éventuellement d'un séchage,
b) un autre recouvrement des particules obtenues dans l'étape a) avec du métal au moyen
b1) d'une décomposition en phase gazeuse de composés métalliques volatils dans une atmosphère inerte ou
b2) d'une libération du métal par voie chimique humide sans courant, suivie éventuellement d'un séchage ou avec un oxyde métallique n'absorbant pas de façon sélective au moyen
b3) d'une décomposition en phase gazeuse de composés métalliques volatils en présence d'oxygène et/ou de vapeur d'eau et
c) un éventuel recouvrement supplémentaire des particules obtenues dans l'étape b) avec un oxyde métallique incolore ou absorbant de façon sélective, par
c1) décomposition en phase gazeuse de composés métalliques volatils en présence d'oxygène et/ou de vapeur d'eau ou
c2) recouvrement par voie chimique humide au moyen d'une décomposition par hydrolyse de composés organométalliques dans lesquels les restes organiques sont reliés aux métaux par des atomes d'oxygène, en présence d'un solvant organique dans lequel les composés métalliques sont solubles, suivi éventuellement d'un séchage.

2. Pigments brillants selon la revendication 1 pour lesquels la couche (B) est constituée pour l'essentiel de chrome, de molybdène, de tungstène, de fer, de cobalt, de nickel, d'argent, de cuivre, d'or, de palladium, de platine, d'alliages du nickel, d'alliages du cobalt, d'alliages argent/or, de magnétite, d'oxyde de nickel, d'oxyde de cobalt et/ou d'oxyde de vanadium.

3. Pigments brillants selon la revendication 1 ou 2 pour lesquels la couche (C) est constituée pour l'essentiel d'oxyde de silicium, d'oxyde de silicium hydraté, d'oxyde d'aluminium, d'oxyde d'aluminium hydraté, d'oxyde de titane, d'oxyde de zirconium, d'oxyde de chrome et/ou d'oxyde de fer.

4. Pigments brillants selon l'une quelconque des revendications 1 à 3 pour lesquels le substrat métallique est constitué pour l'essentiel de plaquettes d'aluminium pouvant être passivées.

5. Pigments brillants à base de substrats à revêtements multiples sous forme de plaquettes constitués pour l'essentiel d'aluminium avec
A) une première couche de 50-600 nm d'épaisseur constituée essentiellement d'oxyde de silicium et/ ou d'oxyde de silicium hydraté,
B) une deuxième couche de 1-25 nm d'épaisseur constituée essentiellement de molybdène, de chrome, de tungstène et/ou de fer et
C) une éventuelle troisième couche de 5-250 nm d'épaisseur constituée essentiellement d'oxyde de silicium, d'oxyde de silicium hydraté, d'oxyde d'aluminium et/ou d'oxyde d'aluminium hydraté.

6. Mélanges de pigments brillants à base
I) de pigments brillants selon l'une quelconque des revendications 1 à 5 et
II) de plaquettes silicatées à revêtements multiples, qui présentent
A') une première couche constituée essentiellement d'oxyde métallique incolore ou absorbant de façon sélective,
B') une deuxième couche constituée essentiellement de métal et/ou d'oxyde métallique n'absorbant pas de façon sélective et
C') une éventuelle troisième couche constituée essentiellement d'oxyde métallique incolore ou absorbant de façon sélective,
en tant que constituants essentiels.

7. Procédé de préparation de pigments brillants à base de substrats métalliques sous forme de plaquettes à revêtements multiples, caractérisé en ce que l'on procède selon les étapes du procédé définies dans la revendication 1.

8. Utilisation de pigments brillants selon l'une quelconque des revendications 1 à 6 pour la coloration de laques, d'encres d'impression, d'encres, de matières plastiques, de verres, de produits céramiques et de préparations pour la cosmétique décorative.
